# EUROPÄISCHE PATENTSCHRIFT

(11) **EP 4 327 747 B1**
(45) Veröffentlichungstag und Bekanntmachung des Hinweises auf die Patenterteilung: **10.12.2025**
(21) Anmeldenummer: 22192175.2
(22) Anmeldetag: 25.08.2022
(51) Int. Cl.: A61B 6/00

(54) **COMPUTERIMPLEMENTIERTES VERFAHREN ZUM BETRIEB EINER RÖNTGENEINRICHTUNG UND RÖNTGENEINRICHTUNG**
COMPUTER-IMPLEMENTED METHOD FOR OPERATING AN X-RAY DEVICE AND X-RAY DEVICE
PROCÉDÉ MIS EN OEVRE PAR ORDINATEUR POUR FAIRE FONCTIONNER UN DISPOSITIF À RAYONS X ET DISPOSITIF À RAYONS X

(43) Veröffentlichungstag der Anmeldung: 28.02.2024
(73) Patentinhaber: Siemens Healthineers AG, 91301 Forchheim (DE)
(72) Erfinder: Meyer, Michael, 91353 Hausen (DE); Pöllath, Michael, 95508 Kulmain (DE); Schmidt, Stefan, 92721 Störnstein (DE)
(74) Vertreter: Siemens Healthineers Patent Attorneys

(56) Entgegenhaltungen:
- EP-A1- 3 620 110
- DE-A1- 102018 107 442
- DE-A1- 19 933 229
- DE-T2- 69 010 033

## Beschreibung

Die Erfindung betrifft ein computerimplementiertes Verfahren zum Betrieb einer Röntgeneinrichtung zur Aufnahme von Projektionsbildern eines Aufnahmebereichs eines Patienten, wobei die Röntgeneinrichtung ein Stativ, einen Trägerarm, eine Halterung und einen C-Bogen, an dem sich gegenüberliegend ein Röntgenstrahler und ein Röntgendetektor angeordnet sind, aufweist, wobei der Trägerarm an einem Ende zur Herstellung eines ersten Bewegungsfreiheitsgrades drehbar um eine erste Rotationsachse mit dem Stativ gekoppelt ist und zur Herstellung eines zweiten Bewegungsfreiheitsgrades die Halterung drehbar um eine zweite Rotationsachse an dem anderen Ende an den Trägerarm gekoppelt ist, so dass sich die erste und die zweite Rotationsachse sowie ein Zentralstrahl des Röntgenstrahlers in allen Lagen des Trägerarms und der Halterung in einem Punkt schneiden, und wobei ferner der gebogene C-Bogen in einer Führung der Halterung zur Herstellung eines dritten Bewegungsfreiheitsgrades verschiebbar gelagert ist, wobei jedem der Bewegungsfreiheitsgrade ein durch eine Steuereinrichtung der Röntgeneinrichtung ansteuerbarer Aktor zugeordnet ist. Daneben betrifft die Erfindung eine Röntgeneinrichtung.

Gerade im Umfeld von medizinischen Interventionen, beispielsweise minimalinvasiven oder sonstigen Eingriffen, besteht der Wunsch nach Röntgeneinrichtungen, die zum einen wenig Platz beanspruchen und anwesende Personen möglichst wenig stören, zum anderen jedoch eine möglichst große Leistungsfähigkeit bereitstellen, nicht nur was die Bildqualität, sondern auch die Anwendungen und Bildaufnahmetechniken betrifft. So besteht insbesondere der Wunsch, mit Röntgeneinrichtungen, die eine im Raum bewegliche Aufnahmeanordnung aus Röntgenstrahler und Röntgendetektor aufweisen, auch dreidimensionale Scans eines Aufnahmebereichs des Patienten durchzuführen. Hierzu werden üblicherweise Projektionsbilder des Aufnahmebereichs in unterschiedlichen Aufnahmegeometrien, insbesondere unter unterschiedlichen Projektionswinkeln, aufgenommen, indem mit der Aufnahmeanordnung geeignete Positionen angefahren werden. Aus den Projektionsbildern kann dann ein dreidimensionaler Bilddatensatz des Aufnahmebereichs in der Art eines Computertomographiebilddatensatzes ermittelt werden, indem ein Rekonstruktionsverfahren eingesetzt wird. Bekannte Rekonstruktionsverfahren umfassen beispielsweise die gefilterte Rückprojektion sowie algebraische/iterative Rekonstruktionstechniken.

Ein anderes Ziel neben der entsprechenden Leistungsfähigkeit ist bei derartigen Röntgeneinrichtungen eine kompakte Ausgestaltung, um Personen im Raum, beispielsweise bei einer medizinischen Intervention und/oder Untersuchung, möglichst wenig zu behindern, zum anderen aber auch eine kostengünstige, wenig komplexe und aufwandsarme, insbesondere kostengünstige Realisierung. Hier sind im Stand der Technik hauptsächlich Röntgeneinrichtungen mit einem C-Bogen bekannt, an dem sich gegenüberliegend der Röntgenstrahler und der Röntgendetektor angeordnet sind. Dabei soll der C-Bogen so kurz wie möglich gehalten werden, um Behinderungen des Personals zu verhindern. Dies führt jedoch dazu, dass beispielsweise für einen 3D-Scan aus der Kopfstellung heraus, also bei in Längsrichtung des Patienten hinter dem Kopf des Patienten angeordnetem C-Bogen, wobei dann die Längsachse des Patienten insbesondere der Rotationsachse des C-Bogens entspricht, nur bestimmte Aufnahmebereiche des Patienten, beispielsweise im Kopf beziehungsweise im oberen Torso überhaupt erreichbar sind. Dies gilt entsprechend bei einer fußseitigen Anordnung. Bestimmte Aufnahmebereiche sind für einen dreidimensionalen Scan mithin nicht erreichbar. Hierbei ist es ein wesentliches Problem, dass es für eine vollständige, hinreichend qualitative Rekonstruktion aus dem Projektionsbildern notwendig ist, einen Projektionswinkelbereich, insbesondere in einer Referenzebene mittels einer (Teil-)Kreisbahn, von mehr als 180° abzudecken. Bei einer gedachten seitlichen Anordnung des C-Bogens, wenn dieser also sozusagen den Patienten beziehungsweise dessen Längsachse umgreift, würde der Versuch, einen derart großen Projektionswinkelbereich abzudecken, jedoch zu Kollisionen mit dem Patiententisch, anderen Komponenten der Röntgeneinrichtung und/oder sogar mit dem Patienten selber führen.

Die Druckschrift DE 10 2018 107 442 A1 zeigt ein Röntgenbildgebungssystem umfassend eine Röntgenstrahlungsquelle, einen Röntgendetektor und einen C-Bogen. Der C-Bogen weist die Röntgenstrahlungsquelle, die an einem ersten Ende angeordnet ist, und den Röntgendetektor auf, der an einem dem ersten Ende gegenüberliegenden zweiten Ende angeordnet ist. Das Röntgenbildgebungssystem enthält ferner ein motorisiertes System, das konfiguriert ist, um den C-Bogen um drei verschiedene Drehachsen herum zu drehen.

DE 690 100 33 T2 betrifft einen Röntgenuntersuchungsapparat mit einer vertikalen Säule, an der eine um eine Horizontalachse drehbarer Arm befestigt ist, an dem ein Träger mit einer Röntgenquelle an einem ersten Ende und einem der Röntgenquelle gegenüber angeordneten Röntgendetektor an einem zweiten Ende befestigt ist.

Aus EP 3 620 110 Al ist ein Röntgengerät bekannt, umfassend eine Röntgenquelle, einen Detektor und eine Trägerstruktur mit einer drehbaren Basis. Der C-Bogen, an dem Quelle und Detektor montiert sind, ist verschiebbar an der Basis befestigt. Die Trägerstruktur ermöglicht zwei Bewegungsfreiheiten: eine Rotation der Basis um eine vertikale Achse und eine Bewegung des C-Bogens entlang eines Bogens.

DE 199 33 229 A1 offenbart beispielhaft eine Röntgeneinrichtung mit einem Röntgenuntersuchungsstativ, welches ein Fundament und einen Arm, dessen erstes Ende um eine im Fundament angeordnete erste Welle drehbar angeordnet ist und dessen zweites Ende mit einer Halterung verbunden ist, in der ein gebogener Träger verschiebbar angeordnet ist. Das eine Ende des Trägers ist mit einer Röntgenröhre und dessen anderes Ende mit einem Rezeptor, die gegeneinander gerichtet sind, versehen. Dabei soll die Halterung des Trägers über eine zweite Welle mit dem Arm drehbar verbunden sein, wobei die erste Welle für das Fundament beziehungsweise für den Arm und die zweite Welle für den Arm beziehungsweise für die Halterung derart gerichtet sind, dass deren fiktive Wellenverlängerungen sowie der Zentralstrahl der Röntgenröhre beziehungsweise des Rezeptors in allen Lagen des Armes und des Trägers einen gemeinsamen Punkt schneiden. Auf diese Weise sollen einzelne, gewünschte Spezialpositionen des einem C-Bogen entsprechenden Trägers eingenommen werden können, wobei es dort konkret möglich sein soll, den Träger von einer kopfgestellten Lage in eine vertikale Seitenlage und/oder in eine laterale Position mit beibehaltenem fiktiven Isozentrum zu bringen. Hierbei soll außerdem der Arzt einen sehr guten Zugang zum Patienten haben. Dennoch ist auch bei einer solchen Röntgeneinrichtung keine Aufnahmetrajektorie entlang einer Kreisbahn in einer Referenzebene, die senkrecht zur Längsrichtung des Patienten steht, bei seitlich angeordneten C-Bogen möglich, ohne dass Kollisionen mit anderen Komponenten und/oder dem Patienten drohen.

Um diese Problematik zu lösen, wurden im Stand der Technik bereits teleskopische Lösungen eingesetzt, die baulich hochkomplex und nur äußerst teuer zu realisieren sind. Auch die vorgeschlagene Verwendung offener Profile, bei denen beispielsweise Rollen, auf denen der C-Bogen geführt ist, während der Untersuchung freiliegen, wurden vorgeschlagen. Diese sind jedoch nachteilhaft, da dann die Mechanik der Röntgeneinrichtung während der Untersuchung frei zugänglich wäre. Dies ist, insbesondere in Umgebungen, in denen medizinische Interventionen und/oder Untersuchungen durchgeführt werden sollen, aus Gründen der Hygiene, Reinigbarkeit und Anfälligkeit der Mechanik unerwünscht.

Der Erfindung liegt die Aufgabe zugrunde, bei einfacher baulicher Ausgestaltung und kompakter Baugröße einer Röntgeneinrichtung kollisionsfrei auch laterale 3D-Scans zu ermöglichen.

Zur Lösung dieser Aufgabe sind erfindungsgemäß ein computerimplementiertes Verfahren mit den Merkmalen des Anspruchs 1 und eine Röntgeneinrichtung mit den Merkmalen des Anspruchs 13 vorgesehen. Vorteilhafte Ausgestaltungen ergeben sich aus den Unteransprüchen.

Bei einem computerimplementierten Verfahren der eingangs genannten Art ist erfindungsgemäß vorgesehen, dass die Aufnahme der Projektionsbilder entlang einer den Patienten bezüglich seiner Längsrichtung lateral umgreifenden, bezüglich einer Referenzebene dreidimensionalen Lateraltrajektorie des Röntgenstrahlers zur Abdeckung eines Projektionswinkelbereichs von wenigstens 200° bezüglich der Referenzebene erfolgt, wobei die Trajektorie unter Ausnutzung aller drei Bewegungsfreiheitsgrade so realisiert wird, dass der C-Bogen entlang der gesamten Lateraltrajektorie bezüglich der Längsrichtung des Patienten oder einer Patientenliege, auf der der Patient positioniert ist, lateral neben dem Patienten positioniert ist.

Konkret steuert die Steuereinrichtung die Aktoren mithin zur Umsetzung der Lateraltrajektorie an, insbesondere zur Herstellung eines bestimmten Bewegungsablaufs bezüglich der Bewegungsfreiheitsgrade, wobei auch seitens der Röntgeneinrichtung die Aufnahme von Projektionsbildern entlang der Lateraltrajektorie gesteuert wird. Aus den Projektionsbildern kann dann, beispielsweise mittels einer Rekonstruktionseinheit der Steuereinrichtung, ein dreidimensionaler Bilddatensatz des Aufnahmebereichs rekonstruiert werden. Mit anderen Worten wird also ein lateraler, dreidimensionaler Scan des Aufnahmebereichs des Patienten ermöglicht.

Während üblicherweise ein dreidimensionaler Scan derart erfolgt, dass als Trajektorie innerhalb einer Referenzebene, insbesondere einer senkrecht zur Längsrichtung des Patienten oder des Patiententisches angeordneten Referenzebene, eine Kreisbahn beziehungsweise Teilkreisbahn genutzt wird, wurde im Rahmen der vorliegenden Erfindung erkannt, dass in einem gewissen Rahmen Abweichungen der Lateraltrajektorie aus der Referenzebene, mithin eine Dreidimensionalität bezüglich der Referenzebene, als zusätzlicher Freiheitsgrad zugelassen werden kann, um trotz einfacher und kostengünstiger Ausstattung der entsprechenden Röntgeneinrichtung einen entsprechenden dreidimensionalen Lateralscan durchführen zu können, ohne Kollisionen befürchten zu müssen oder die Erreichbarkeit bestimmter Einstellungen entlang der Kreisbahn aufgrund der Mechanik, mit der die Aufnahmeanordnung gehaltert ist, zwangsläufig bereitstellen zu müssen. Anders gesagt erlaubt ein Verlassen der Referenzebene durch die Lateraltrajektorie die Erweiterung des Verfahrbereichs, ohne dass komplexe Anpassungen an der Mechanik, den Aktoren und den Bewegungsfreiheitsgraden vorzunehmen sind. Es kann also gesagt werden, dass erfindungsgemäß die dreidimensionale Lateraltrajektorie zumindest nicht vollständig in einer Referenzebene verläuft, sondern aus dieser wenigstens teilweise abweicht.

In diesem Kontext kann der Projektionswinkel also als der Winkel der Projektion des Zentralstrahls auf die Referenzebene verstanden werden, da es im abzudeckenden Projektionswinkelbereich um eben diese Referenz geht.

Durch die laterale Positionierung des C-Bogens bezüglich der Längsrichtung des Patienten beziehungsweise der Patientenliege umgreift dieser den Patienten seitlich, so dass der Aufnahmebereich im Sichtfeld der aus Röntgenstrahler und Röntgendetektor gebildeten Aufnahmeanordnung liegt. Dabei ist auch ein solches Umgreifen von unten und oben bei liegendem Patienten in Bezug auf die Längsrichtung des Patienten und/oder der Patientenliege als laterale Positionierung zu verstehen.

Durch die Abweichung der Lateraltrajektorie aus der Referenzebene ist es mithin möglich, den Verfahrbereich so zu erweitern, dass ein hinreichender Projektionswinkelbereich derart abgedeckt wird, dass aus den Projektionsbildern ein dreidimensionaler Bilddatensatz des Aufnahmebereichs, der eine hinreichende Qualität aufweist, rekonstruiert werden kann. Es wird ein weiterer Freiheitsgrad für die Trajektorienplanung eröffnet, nämlich das Verlassen der Referenzebene, um hinreichende Projektionswinkelbereiche bezüglich der Referenzebene trotz gegebenenfalls vorhandener Einschränkungen der Bewegungsfreiheitsgrade und/oder ohne Kollisionsgefahr mit dem Patienten oder einer Komponente der Röntgeneinrichtung zu erreichen.

Dabei kann insbesondere vorgesehen sein, dass ausschließlich die drei genannten Bewegungsfreiheitsgrade (Rotation um erste und zweite Rotationsachse sowie Verschieben des C-Bogens in der Halterung) zur Verstellung des C-Bogens entlang der Lateraltrajektorie verwendet werden, indem die entsprechenden Aktoren durch die Steuereinrichtung entsprechend angesteuert werden. Dabei wurde im Rahmen der vorliegenden Erfindung nämlich insbesondere erkannt, dass beispielsweise mittels einer wie in DE 199 33 229 A1 beschriebenen Röntgeneinrichtung mit den drei einfach realisierbaren, nicht offenliegenden Freiheitsgraden bereits durch Erlauben einer Abweichung aus der Referenzebene, mithin einer dreidimensionalen Lateraltrajektorie, hochqualitative dreidimensionale Bilddatensätze von Aufnahmebereichen erhalten werden können. Während der zweite und der dritte Bewegungsfreiheitsgrad, Rotation um die zweite Rotationsachse sowie Verschieben in der Halterung (sogenannte Orbitaldrehung) im Stand der Technik bereits grundsätzlich bekannt sind, ist es also im Rahmen der vorliegenden Erfindung nun insbesondere möglich, durch Hinzufügen des Trägerarms sowie des ersten Bewegungsfreiheitsgrades, was einfach und ohne freiliegende Mechanik möglich ist, bereits alles bereitzustellen, was nötig ist, um einen lateralen dreidimensionalen Scann eines Aufnahmebereichs zu ermöglichen, indem mögliche Unzulänglichkeiten der Bewegungsmechanik hinsichtlich anfahrbarer Positionen und/oder das Risiko von Kollisionen dadurch umgangen werden, dass die Lateraltrajektorie dreidimensional ausgestaltet wird, mithin aus einer Referenzebene, in der sie beispielsweise idealerweise als eine Teilkreisbahn vorgesehen war, herausführen kann.

Allgemein kann gesagt werden, dass es sich bei der vorliegenden Realisierung vorteilhafterweise weder um ein komplexes teleskopisches Prinzip noch um ein Prinzip, bei welchem die Laufrollen die Führung verlassen müssen, handelt. Durch die geschickte Anordnung der Bewegungsfreiheitsgrade kann ein Verfahrwinkel von mehr als 180° realisiert werden und es bietet sich die Möglichkeit, laterale dreidimensionale Aufnahmen von Aufnahmebereichen eines Patienten zu machen. Bisher war dies mit einer derartigen Röntgeneinrichtung nur in der Kopfstellung ("Headside") möglich.

Dabei sei an dieser Stelle darauf hingewiesen, dass in der kopfseitigen Stellung, wenn also die zweite Rotationsachse zumindest im Wesentlichen parallel zur Längsrichtung des Patienten und/oder der Patientenliege ist, mit der Röntgeneinrichtung selbstverständlich auch, wie bekannt, weiterhin dreidimensionale Scans durchgeführt werden können. In diesem Sinne kann also gesagt werden, dass die Röntgeneinrichtung um eine weitere Aufnahmemöglichkeit erweitert wird.

In zweckmäßiger Weiterbildung der vorliegenden Erfindung kann vorgesehen sein, dass die Lateraltrajektorie in einem Optimierungsverfahren unter Einhaltung eines Sicherheitsabstands zu dem Patienten und/oder zu Komponenten der Röntgeneinrichtung als Randbedingung so ermittelt wird, dass sie möglichst nahe an einer Idealtrajektorie in der Referenzebene zu liegen kommt. Das bedeutet, um möglichst nahe an einer Idealtrajektorie in der Referenzebene bleiben zu können, können die Aktoren der Bewegungsfreiheitsgrade derart abgestimmt aufeinander betrieben werden, dass die Einstellmöglichkeiten optimal ausgenutzt werden, ohne dass es dabei zu Kollisionen, sei es mit dem Patienten oder mit Komponenten der Röntgeneinrichtung, kommt. Dabei können grundsätzlich bekannte Optimierungstechniken herangezogen werden, die die Möglichkeiten der verschiedenen Bewegungsfreiheitsgrade entsprechend berücksichtigen. Hierbei kann, wie bereits erwähnt, die Idealtrajektorie eine Teilkreisbahn sein, nachdem derartige Teilkreisbahnen, um den Projektionswinkelbereich in der Referenzebene abzudecken, im Stand der Technik häufig eingesetzt werden. Auf Basis solcher Teilkreisbahnen ist eine besonders einfache Rekonstruktion eines dreidimensionalen Bilddatensatzes aus den zweidimensionalen Projektionsbildern möglich, welche sich bei leichten Abweichungen aus der Referenzebene auch auf die entlang der dreidimensionalen Lateraltrajektorie aufgenommenen Projektionsbilder zumindest teilweise anwenden oder übertragen lässt. Bei der Referenzebene kann es sich um eine vertikale, insbesondere zur Längsrichtung des Patienten und/oder der Patientenliege senkrechte Ebene handeln, mithin insbesondere um eine Transversalebene, nachdem dies einen medizinischen Standard darstellt, der auch bei Kopfstellung üblicherweise verwendet wird.

Als weitere Randbedingung kann wenigstens eine maximale Winkelabweichung des Zentralstrahls aus der Referenzebene, insbesondere von 10 bis 20°, bevorzugt 15°, und/oder als Optimierungsziel eine Minimierung dieser Winkelabweichung vorgegeben werden. Es kann also ein grundsätzliches Ziel sein, die Winkelabweichung aus der Referenzebene gering zu erhalten. Auf diese Weise wird auch der Einfluss der Dreidimensionalität auf die Rekonstruktion möglichst gering gehalten, so dass aufgrund der Dreidimensionalität der Lateraltrajektorie entstehende Artefakte/Bildqualitätsstörungen auch ebenso gering gehalten werden können. Dabei hat sich gezeigt, dass bei einer Beschränkung der Winkelabweichungen auf kleiner 15°, insbesondere höchstens 12°, bereits qualitativ äußerst hochwertige Projektionsbildersätze und somit dreidimensionale Bilddatensätze erhalten werden können.

In einer besonders zweckmäßigen Ausgestaltung des Verfahrens kann vorgesehen sein, dass eine patientenspezifische Ausdehnungsinformation ermittelt und bei der Optimierung zur Auswertung der Randbedingungen berücksichtigt wird. Im Stand der Technik wurden bereits eine Vielzahl von Möglichkeiten vorgeschlagen, Patientengrößen beziehungsweise Ausdehnungen in verschiedenen Konkretisierungsstufen zu ermitteln und zu berücksichtigen. So kann beispielsweise eine Patienteninformation in einem einfachen Fall lediglich die Lage des Patienten auf der Patientenliege gemeinsam mit allgemeinen Patientendaten, wie Alter, Geschlecht, Größe und/oder Gewicht, umfassen. Besonders vorteilhaft ist es jedoch, wenn die patientenspezifische Ausdehnungsinformation durch eine Messung bestimmt wird. Beispielsweise kann eine Abtastung der Patientenoberfläche durch eine 3D-Kamera, insbesondere eine Terahertz-Kamera, und/oder ein Radargerät erfolgen. Möglich ist es auch, Übersichtsbilddaten mittels der Röntgeneinrichtung selbst aufzunehmen und entsprechend auszuwerten. In allen diesen Fällen ist es möglich, sich patientenindividuell ergebende Freiräume bei der Trajektorienplanung verbessert auszunutzen und somit gegebenenfalls näher an die Idealtrajektorie heranzukommen, gleichzeitig aber auch insbesondere voluminösere Patienten verbessert vor Kollisionen zu schützen.

Vorzugsweise kann der Projektionswinkelbereich über einen abzudeckenden Referenzwinkelbereich, insbesondere von 180° plus Fächerwinkel, beispielsweise 200°, hinaus zur Aufnahme weiterer Projektionsbilder zur Reduzierung von Artefakten bei einer nachfolgenden Rekonstruktion eines dreidimensionalen Bilddatensatzes erweitert werden. Es hat sich gezeigt, dass es durch die Abweichungen aus der Referenzebene bei der Rekonstruktion, insbesondere wenn diese unverändert übernommen wird, zu Artefakten und/oder Bildqualitätsstörungen kommen kann. Durch die Aufnahme zusätzlicher Projektionsbilder unter weiteren Projektionswinkeln, mithin eine Erweiterung des Projektionswinkelbereichs gegenüber einem Referenzwinkelbereich, ist es möglich, Zusatzinformationen bereitzustellen, die mithelfen, solche Artefakte und/oder Bildqualitätsstörungen deutlich zu reduzieren und eine hervorragende Bildqualität des rekonstruierten dreidimensionalen Bilddatensatzes herzustellen. Dabei hat sich bei Untersuchungen gezeigt, dass schon durch eine kleinere Erweiterung des Projektionswinkelbereichs gegenüber dem Referenzwinkelbereich starke Qualitätsgewinne verzeichnet werden können. Beispielsweise kann vorgesehen sein, dass der Projektionswinkelbereich gegenüber dem Referenzwinkelbereich um 2 bis 10°, insbesondere 5 bis 7°, erweitert wird. Statt einem Referenzwinkelbereich von 200° ist es also beispielsweise denkbar, einen Projektionswinkelbereich von 206° abzudecken, was beispielsweise bei einer maximalen Winkelabweichung aus der Referenzebene von 12° zu qualitativ hervorragenden Bildergebnissen führt.

Vorzugsweise kann vorgesehen sein, dass die erste Rotationsachse um 1 bis 20°, insbesondere 18°, gegenüber der Horizontalen geneigt ist und/oder der Verfahrbereich um die erste Rotationsachse 140 bis 160°, insbesondere 150°, und/oder der Verfahrbereich um die zweite Rotationsachse 300 bis 320°, insbesondere 310°, und/oder der Verfahrbereich durch die Verschiebung des C-Bogens in der Halterung 140 bis 160°, insbesondere 150°, beträgt. Untersuchungen haben gezeigt, dass sich mit diesen Werten ideale Randbedingungen ergeben, um die Abweichung aus der Referenzebene bzw. von einer Idealtrajektorie für eine aufrechterhaltene hohe Bildgebungsqualität auch für den lateralen Scan möglichst gering zu halten. Eine hervorragende Auslegung ergibt sich bei einer Schrägstellung der ersten Rotationsachse um 18°, Verfahrbereichen von 150° für die erste Rotationsachse und die Verschiebung und einem Verfahrbereich von 310° für die zweite Rotationsachse.

Eine Weiterbildung des Verfahrens sieht vor, dass der Röntgendetektor mittels eines weiteren, durch die Steuereinrichtung ansteuerbaren Aktors um eine zu dem Zentralstrahl parallele, insbesondere diesem entsprechende, Detektorrotationsachse drehbar gelagert ist, wobei während der Lateraltrajektorie die Steuereinrichtung den weiteren Aktor so ansteuert, dass der Röntgendetektor eine vorbestimmte Orientierung, insbesondere mit gegenüberliegenden Längskanten entlang einer Längsrichtung der Patientenliege, für alle Projektionsbilder einnimmt. Auf diese Weise kann sichergestellt werden, dass auch bei insbesondere aus der Referenzebene abweichenden Trajektorienanteilen die Bilder eine gewünschte, vorgegebene Orientierung haben, so dass ein aufwendiges Umrechnen entlang der Lateraltrajektorie vorteilhaft entfallen kann. Zudem kann Vorgaben beziehungsweise Standards gefolgt werden.

Der Aufnahmebereich kann beispielsweise ein Abdomenbereich und/oder ein Hüftbereich sein, insbesondere eine Leber des Patienten umfassen. Wie bereits erwähnt, ist es eine allgemeine Auslegungsbestrebung, um die Röntgeneinrichtungen kompakt und möglichst wenig störend für andere Personen zu halten, den C-Bogen eher kurz auszugestalten, so dass die Nutzbarkeit des C-Bogens für dreidimensionale Scans in einer Kopfstellung häufig auf den Kopf und gegebenenfalls Anteile des oberen Torsos eingeschränkt ist. Das bedeutet, mit solchen Röntgeneinrichtungen war es bislang nicht ohne großen Aufwand und Umgestaltung desselben möglich, dazwischen liegende Aufnahmebereiche sinnvoll dreidimensional zu erfassen, beispielsweise die Leber, den Hüftbereich und gegebenenfalls auch Bereiche des Oberschenkels, beispielsweise bei einem Bruch des Oberschenkelknochens. Mit der hier beschriebenen Option eines lateralen dreidimensionalen Scans entlang der Lateraltrajektorie wird nun auch bei derartigen Röntgeneinrichtungen die Möglichkeit eröffnet, solche Aufnahmebereiche zu erfassen.

Vorteilhafterweise kann das Stativ bodenmontiert sein. Eine bodenseitige Installation ist deutlich einfacher konstruktiv umzusetzen und stellt deutlich geringere Anforderungen an die entsprechenden Befestigungsmittel; zudem wird weniger relevanter Bauraum beziehungsweise Sichtfeld weggenommen. Grundsätzlich ist es im Rahmen der vorliegenden Erfindung jedoch auch denkbar, ein deckenmontiertes Stativ vorzusehen, jedoch weniger bevorzugt.

Aufgrund der robusten, einfach umgesetzten Mechanik, insbesondere bei Nutzung ausschließlich der drei genannten Freiheitsgrade, sind auf stabile Art und Weise relativ hohe Bewegungsgeschwindigkeiten der Aufnahmeanordnung, insbesondere des Röntgenstrahlers, entlang der Lateraltrajektorie möglich.

Beispielsweise kann die Bewegung entlang der Lateraltrajektorie mit einer Winkelgeschwindigkeit von wenigstens 40° pro Sekunde, insbesondere wenigstens 50° pro Sekunde, erfolgen. Dabei kann sich die Winkelgeschwindigkeit insbesondere auch auf den Projektionswinkel in der Referenzebene beziehen. Dies ermöglicht äußerst schnelle Akquisitionen, nachdem der Projektionswinkelbereich in wenigen Sekunden vollständig abgetastet ist. Damit ist auch die Anfälligkeit gegenüber Bewegungen deutlich reduziert.

Gerade in diesem Zusammenhang ist anzumerken, dass die erfindungsgemäße Vorgehensweise auch für Bildaufnahmetechniken, die Kontrastmittel nutzen, eingesetzt werden kann, da hinreichend schnelle dreidimensionale laterale Scans unter Nutzung der dreidimensionalen, wenigstens teilweise aus der Referenzebene abweichenden Lateraltrajektorie möglich sind. Dies gilt insbesondere für die digitale Subtraktionsangiographie (DSA), bei der mit besonderem Vorteil vorgesehen sein kann, dass Projektionsbilder während zwei aufeinanderfolgenden, in umgekehrten Richtungen erfolgenden Durchläufen der Lateraltrajektorie aufgenommen werden, insbesondere als Masken-Projektionsbilder vor dem Eintreffen eines Kontrastmittels zum Aufnahmebereich und beim zweiten Durchlauf der Lateraltrajektorie als Füllungsbilder nach Ankunft des Kontrastmittels im Aufnahmebereich. Konkret kann beispielsweise vorgesehen sein, dass zunächst der C-Bogen und somit die Aufnahmeanordnung aus Röntgenstrahler und Röntgendetektor in eine Startposition, die einer der Endpositionen der Lateraltrajektorie entspricht, verbracht wird. Von dort aus erfolgt, insbesondere zeitlich abgestimmt mit einem Kontrastmittelanreicherungsverlauf im Aufnahmebereich, zunächst der erste Durchlauf der Lateraltrajektorie bis zur anderen Endposition der Lateraltrajektorie zur Aufnahme der Masken-Projektionsbilder (Maskenlauf). Von dieser anderen Endposition der Lateraltrajektorie aus erfolgt dann der zweite Durchlauf der Lateraltrajektorie bis zur als Startposition genutzten Endposition der Lateraltrajektorie zurück, wobei dann die Füllungs-Projektionsbilder aufgenommen werden können (Füllungslauf).

Aus der Startposition kann der C-Bogen dann beispielsweise wieder in eine Ruheposition zurückbewegt werden.

Neben dem Verfahren betrifft die Erfindung auch eine Röntgeneinrichtung, aufweisend ein Stativ, einen Trägerarm, eine Halterung und einen C-Bogen, an dem sich gegenüberliegend ein Röntgenstrahler und ein Röntgendetektor angeordnet sind, wobei der Trägerarm an einem Ende zur Herstellung eines ersten Bewegungsfreiheitsgrades drehbar um eine erste Rotationsachse mit dem Stativ und zur Herstellung eines zweiten Bewegungsfreiheitsgrades die Halterung drehbar um eine zweite Rotationsachse an dem anderen Ende an dem Trägerarm angekoppelt ist, so dass sich die erste und die zweite Rotationsachse sowie ein Zentralstrahl des Röntgenstrahlers in allen Lagen des Trägerarms und der Halterung in einem Punkt schneiden, und wobei ferner der C-Bogen in einer Führung der Halterung zur Herstellung eines dritten Bewegungsfreiheitsgrades verschiebbar gelagert ist, wobei zu jedem der Bewegungsfreiheitsgrade ein durch eine Steuereinrichtung der Röntgeneinrichtung ansteuerbarer Aktor zugeordnet ist, welche Röntgeneinrichtung sich dadurch auszeichnet, dass die Steuereinrichtung zur Durchführung des erfindungsgemäßen Verfahrens ausgebildet ist. Sämtliche Ausführungen bezüglich des erfindungsgemäßen Verfahrens lassen sich analog auf die erfindungsgemäße Röntgeneinrichtung übertragen, mit welcher mithin ebenso die bereits genannten Vorteile erhalten werden können.

Die Steuereinrichtung kann wenigstens einen Prozessor und wenigstens ein Speichermittel umfassen. Durch Hardware und/oder Software der Steuereinrichtung können Funktionseinheiten geschaffen werden, um verschiedene Schritte des erfindungsgemäßen Verfahrens durchzuführen. So kann die Steuereinrichtung beispielsweise eine Trajektorieneinheit zur Ansteuerung der Aktoren zum Durchlaufen von Aufnahmetrajektorien, insbesondere der Lateraltrajektorie, genauso aufweisen wie eine Aufnahmeeinheit, die den Aufnahmebetrieb des Röntgenstrahlers und des Röntgendetektors steuert, insbesondere die Aufnahme der Projektionsbilder. In einer Rekonstruktionseinheit können die zweidimensionalen Projektionsbilder genutzt werden, um einen dreidimensionalen Bilddatensatz zu rekonstruieren. Die Steuereinrichtung kann auch weitere Funktionseinheiten zur Umsetzung von Weiterbildungen des erfindungsgemäßen Verfahrens umfassen, beispielsweise eine Trajektorienplanungseinheit zur Durchführung eines Optimierungsverfahrens zur Planung der Lateraltrajektorie.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: eine Prinzipskizze einer erfindungsgemä-ßen Röntgeneinrichtung,
- Fig. 2: einen Ablaufplan eines Ausführungsbeispiels des erfindungsgemäßen Verfahrens,
- Fig. 3 bis Fig. 6: verschiedene Stellungen der Röntgeneinrichtung während einer beispielhaften Lateraltrajektorie,
- Fig. 7: den Verlauf der Position des Röntgendetektors während der Lateraltrajektorie der Fig. 3 bis 6, und
- Fig. 8: den funktionalen Aufbau einer Steuereinrichtung der Röntgeneinrichtung.

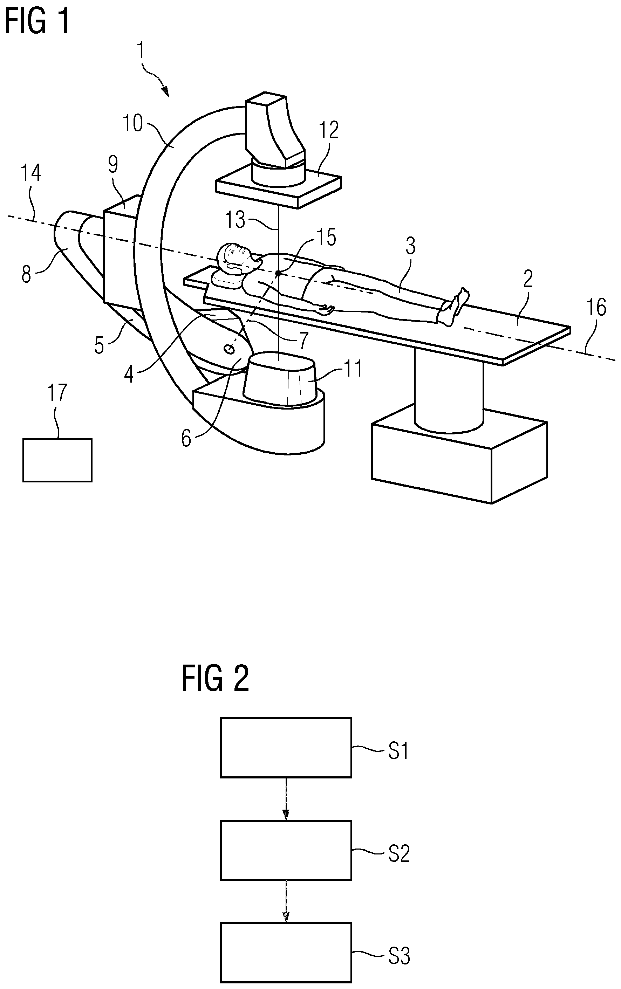

Fig. 1 zeigt eine Prinzipskizze einer erfindungsgemäßen Röntgeneinrichtung 1. Diese umfasst zunächst eine Patientenliege 2, auf der ein Patient 3 angedeutet ist. Die Patientenliege ist Teil eines Patiententisches, der beispielsweise bodenfest ausgeführt sein kann.

Auf dem Boden ist ebenso ein Stativ 4 befestigt, an dem ein Trägerarm 5 an einem ersten Ende 6 drehbar um eine Rotationsachse 7 angeordnet ist. Das zweite Ende 8 des Trägerarms 5 ist mit einer Halterung 9 verbunden, in der ein C-Bogen verschiebbar gelagert ist. Der C-Bogen 10 weist einen Röntgenstrahler 11 und diesen gegenüberliegend einen Röntgendetektor 12 auf, wobei ein Zentralstrahl 13 dieser Aufnahmeanordnung ebenso gezeigt ist. Die Halterung 9 ist an dem zweiten Ende 8 des Trägerarms 5 um eine zweite Rotationsachse 14 drehbar gelagert. Ersichtlich schneiden sich die erste Rotationsachse 7, die zweite Rotationsachse 14 und der Zentralstrahl 13 in einem gemeinsamen Punkt 15, was für alle Stellungen/Lagen der entsprechenden Komponenten gilt. Hierbei ist unabhängig von der konkreten Einstellung, mithin der Drehung des Trägerarms 5, der Drehung der Halterung 9 und der Verschiebung des C-Bogens 10 die Lage des Punktes 15 im Raum immer gleich.

Die erste Rotationsachse 7 ist dabei um 18° gegenüber der Horizontalen schräggestellt. Die Verfahrbereiche hinsichtlich der ersten Rotationsachse 7 und der Verschiebung des C-Bogens 10 in der Halterung 9 betragen 150° und der Verfahrbereich um die zweite Rotationsachse beträgt 310°.

Fig. 1 zeigt den C-Bogen 10 in einer sogenannten kopfgestellten Lage. Hierbei ist der Patient 3 derart auf der Patientenliege 2 gelagert, dass seine Längsrichtung parallel zu der Längsrichtung 16 der Patientenliege 2 verläuft. In der Kopfstellung der Fig. 1 verläuft nun die zweite Rotationsachse 14 zumindest im Wesentlichen parallel zu dieser Längsrichtung 16. Damit kann der C-Bogen 10 derart um den Patienten 3 gedreht werden, diesen vom Kopf her umgreifend, dass sich der Röntgenstrahler 11 entlang einer Kreisbahn in einer zur Längsrichtung 16 senkrechten Ebene bewegt. Allerdings ist der C-Bogen 10 kurz ausgebildet, so dass von der Kopfstellung her Aufnahmebereiche im Abdomen, Hüftbereich und wenigstens teilweise Oberschenkelbereich auf diese Art und Weise nicht gescannt werden können.

Zur Steuerung des Betriebs der Röntgeneinrichtung 1 weist diese eine Steuereinrichtung 17 auf, mit der nicht nur ein eben beschriebener kopfseitiger Scan durchgeführt werden kann, sondern die Steuereinrichtung 17 ist auch zur Durchführung des erfindungsgemäßen Verfahrens ausgebildet, welches einen lateralen dreidimensionalen Scan unter Nutzung der drei beschriebenen Bewegungsfreiheitsgrade (Drehung um erste Rotationsachse 7, Drehung um zweite Rotationsachse 14 und Verschiebung in der Halterung 9, welche eine entsprechende Führung aufweist) erlaubt.

Dabei sei an dieser Stelle noch angemerkt, dass allen drei diskutierten Bewegungsfreiheitsgraden des C-Bogens 10 entsprechende, in Fig. 1 der Übersichtlichkeit halber nicht näher gezeigte Aktoren zugeordnet sind, die über die Steuereinrichtung 17 ansteuerbar sind. Das bedeutet, sowohl was die Rotation um die erste Rotationsachse 7 und die zweite Rotationsachse 14 angeht als auch was die Verschiebung des C-Bogens 10 in der Führung der Halterung 9 angeht, sind jeweils Aktoren vorhanden die ansteuerbar sind. Eine Trajektorie des Röntgenstrahlers 11 kann insbesondere beschrieben werden durch den Verlauf von Steuersignalen für die Aktoren (und somit entsprechende Teil-Bewegungsabläufe in den Bewegungsfreiheitsgraden). Als weitere, mittels der Steuereinrichtung 17 ansteuerbare Bewegungsmöglichkeit ist der hier als Flachdetektor ausgebildete Röntgendetektor 12 um eine dem Zentralstrahl 13 entsprechende Rotationsachse rotierbar.

Fig. 2 zeigt einen Ablaufplan eines Ausführungsbeispiels des erfindungsgemäßen Verfahrens. Dabei wird eine Lateraltrajektorie bestimmt, die, um ohne Kollision und innerhalb der Bewegungsfreiräume der Bewegungsfreiheitsgrade umsetzbar zu sein, einen zusätzlichen Freiheitsgrad nutzt, und zwar die Möglichkeit, von einer hier vertikalen, senkrecht zur Längsrichtung 16 ausgerichteten Referenzebene abzuweichen, das bedeutet, diese zu verlassen und somit dreidimensional zu sein.

Konkret wird in einem Schritt S1 in einem Optimierungsverfahren zunächst die Lateraltrajektorie bestimmt. Das Optimierungsziel ist dabei, eine Lateraltrajektorie zu erhalten, die möglichst nahe an der Idealtrajektorie in der Referenzebene liegt, hier einer Teilkreisbahn in einer vertikalen, zur Längsrichtung 16 senkrechten Ebene, wie sie beispielsweise in der Kopfstellung durch einfache Drehung um die zweite Rotationsachse 14, vgl. Fig. 1, umgesetzt werden könnte. Ein weiteres Optimierungsziel kann dabei sein, möglichst geringe Winkelabweichungen aus der Referenzebene, beispielsweise bezüglich des Punktes 15, zuzulassen. Als Randbedingungen wird neben den vorgegebenen Bewegungsfreiräumen der einzelnen Bewegungsfreiheitsgrade, mithin dem mechanisch Möglichen, gefordert, dass keine Kollision mit dem Patienten 3 oder einer Komponente der Röntgeneinrichtung 1 stattfindet, insbesondere also nicht mit der Patientenliege 2 beziehungsweise dem diese tragenden Tischständer. Weitere Randbedingungen können umfassen, dass ein bestimmter Abweichungswinkel aus der Referenzebene nicht überschritten werden soll. Die Lateraltrajektorie wird in dem Optimierungsverfahren so bestimmt, dass ein bestimmter Projektionswinkelbereich, der gewünscht ist, abgedeckt wird, wobei sich der Projektionswinkelbereich auf die Referenzebene bezieht, mithin die Projektion des Zentralstrahls in diese. Der Projektionswinkelbereich wird dabei zweckmäßigerweise größer gewählt als das zur vollständigen Rekonstruktion erforderliche Minimum, insbesondere also größer als 180° plus dem Fächerwinkel. Bei einem Fächerwinkel von 20° würden für eine vollständige Rekonstruktion durch Projektionsbilder abgedeckt 200° grundsätzlich ausreichen, jedoch wird hier aus der Referenzebene abgewichen, so dass zusätzliche Projektionsinformationen nützlich sind, um Artefakte und Bildqualitätsstörungen möglichst weitgehend zu reduzieren. Im vorliegenden Fall wird beispielhaft ein gegenüber dem Referenzwinkelbereich von 200° erweiterter Projektionswinkelbereich von 206° durch die Lateraltrajektorie abgedeckt.

Im vorliegenden Ausführungsbeispiel wird die Lateraltrajektorie patientenspezifisch dahingehend bestimmt, dass für die Auswertung der Randbedingung eine patientenspezifische Ausdehnungsinformation herangezogen wird, die auf vorliegenden Patientendaten wie Größe, Geschlecht, Alter und Gewicht basieren kann, bevorzugt jedoch mittels einer Messeinrichtung, beispielsweise einer 3D-Kamera, insbesondere als Terahertz-Kamera, und/oder einer Radareinrichtung vermessen wurden.

In einem Schritt S2 wird dann mittels einer Trajektorieneinheit der Steuereinrichtung die bestimmte Lateraltrajektorie durch Ansteuerung der entsprechenden Aktoren abgefahren. Gleichzeitig werden, gesteuert über eine Aufnahmeeinheit der Steuereinrichtung, mittels der aus dem Röntgenstrahler 11 und dem Röntgendetektor 12 gebildeten Aufnahmeanordnung Projektionsbilder unter unterschiedlichen Projektionsgeometrien, mithin unterschiedlichen Projektionswinkeln, aufgenommen. Der Aufnahmebereich kann dabei insbesondere ein Abdomenbereich und/oder ein Hüftbereich sein, da diese in der Kopfstellung, wie beschrieben, nicht dreidimensional scannbar sind. Die Geschwindigkeit, mit der sich der Röntgenstrahler 11 entlang der Lateraltrajektorie bewegt und die daher auch der Geschwindigkeit des Röntgendetektors 12 entspricht, kann wenigstens 40° pro Sekunde bezogen auf den Projektionswinkel, insbesondere wenigstens 50° pro Sekunde bezogen auf den Projektionswinkel, betragen. So ist eine äußerst schnelle, wenig bewegungsanfällige Messung möglich.

In einem Schritt S3 werden die Projektionsbilder dann ausgewertet, um mittels eines Rekonstruktionsverfahrens einen dreidimensionalen Bilddatensatz des Aufnahmebereichs zu rekonstruieren. Als Rekonstruktionsverfahren kann beispielsweise ein Verfahren der gefilterten Rückprojektion verwendet werden. Der so rekonstruierte dreidimensionale Bilddatensatz kann beispielsweise einem Benutzer zur Anzeige gebracht werden, gespeichert werden oder aber auch weiter ausgewertet werden.

Dabei sei an dieser Stelle noch angemerkt, dass grundsätzlich auch ein zweifaches Durchlaufen der Lateraltrajektorie erfolgen kann, beispielsweise hin und zurück, um sowohl Masken-Projektionsbilder als auch Füllungs-Projektionsbilder bei Verwendung eines Kontrastmittels, insbesondere bei einer Untersuchung der digitalen Subtraktionsangiographie, aufnehmen zu können.

Im Schritt S2 kann die Steuereinrichtung 17 auch ausgebildet sein, einen der Verdrehung des Röntgendetektors 12 zugeordneten Aktor anzusteuern, um, auch bei Abweichung aus der Referenzebene, die Ausrichtung des Detektors so anzupassen, dass eine vorgegebene Orientierung, beispielsweise mit Längskanten entsprechend der Längsrichtung 16, gegeben ist.

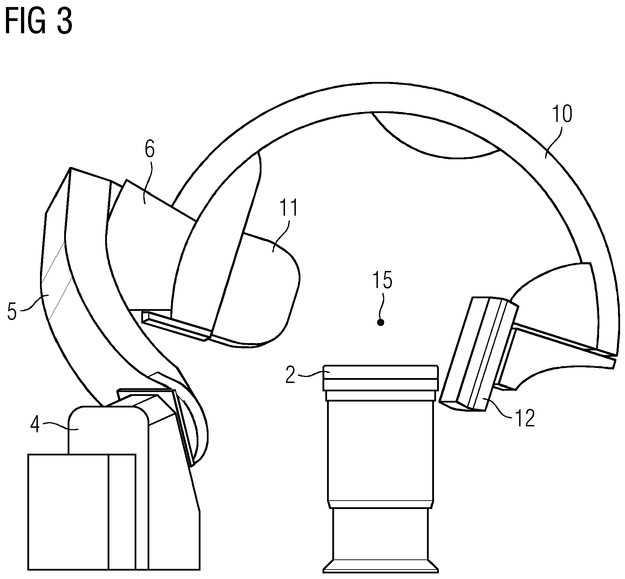

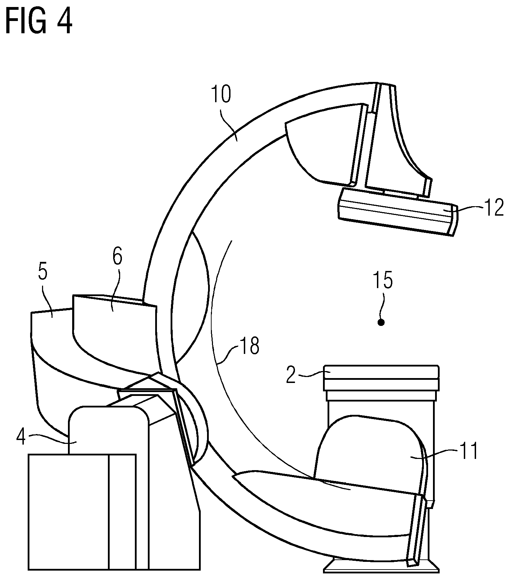

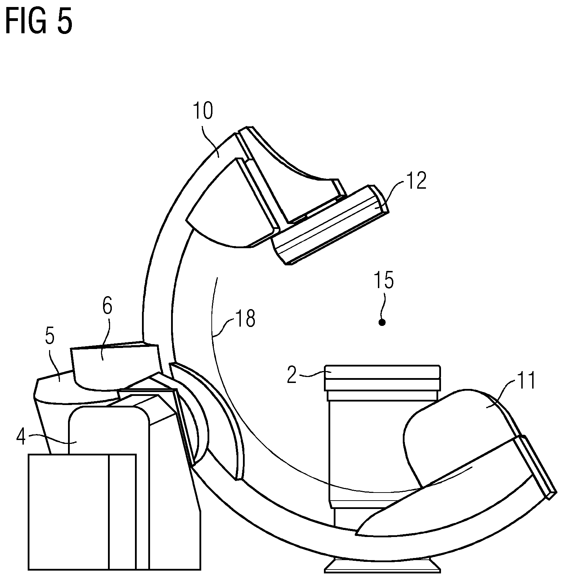

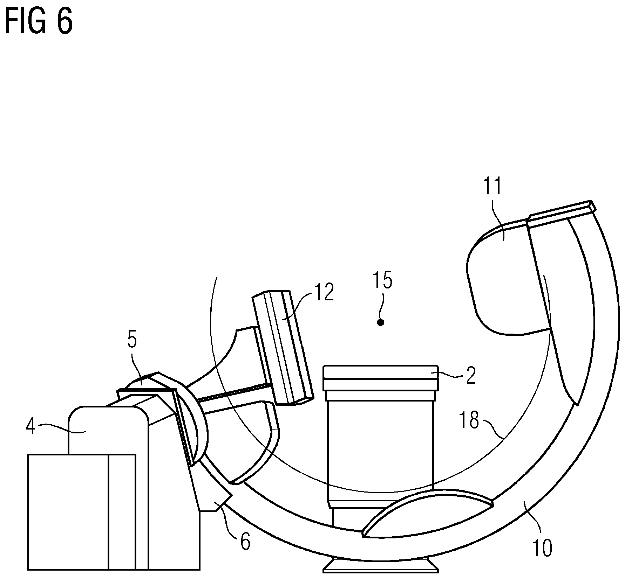

Die Figuren 3 bis 6 zeigen beispielhaft eine mögliche Realisierung der Lateraltrajektorie in Form von vier Momentaufnahmen, wobei Fig. 3 dem Startpunkt als eine erste Endposition der Lateraltrajektorie und Fig. 6 den Endpunkt als zweite Endposition der Lateraltrajektorie zeigt. Die Figuren 4 und 5 zeigen Zwischenzustände. Die sich entwickelnde und langsam vervollständigende Lateraltrajektorie 18 ist entsprechend angedeutet.

Fig. 9 zeigt die Bewegung des Röntgendetektors 12 als absolute Position (Pos.) entlang der Achsen eines rechtwinkligen Koordinatensystems, welches seinen Ursprung im Punkt 15 hat, gegen die Zeit (t) während der Lateraltrajektorie der Fig. 3 bis 6. Dabei bezieht sich die Kurve 24 auf eine der Längsrichtung 16 entsprechende Koordinate, die Kurven 25 und 26 jeweils auf eine dazu senkrechte horizontale und eine dazu senkrechte vertikale Richtung. Die Kurve 24 zeigt deutlich die Abweichung aus der Referenzebene, während die Kurven 24 und 25 die Abdeckung des Projektionswinkelbereichs illustrieren, aber auch die Abweichung von einer perfekten Kreisbahn/Teilkreisbahn zeigen.

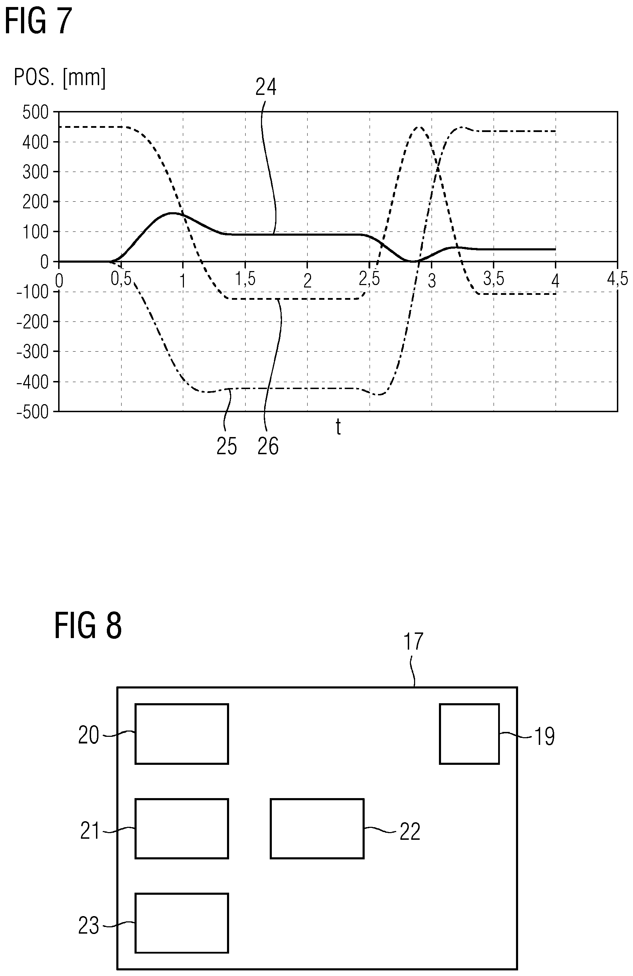

Fig. 8 zeigt den funktionalen Aufbau der zur Durchführung des erfindungsgemäßen Verfahrens ausgebildeten Steuereinrichtung 17 genauer. Dieser weist zunächst ein Speichermittel 19 auf, in dem Zwischen- und Endergebnisse gespeichert werden können, beispielsweise die Projektionsbilder und der dreidimensionale Bilddatensatz.

In einer Trajektorienplanungseinheit 20 wird die Lateraltrajektorie gemäß Schritt S1 bestimmt. Eine Trajektorieneinheit 21 zur Ansteuerung der Aktoren und eine Aufnahmeeinheit 22 zur Ansteuerung der Aufnahmeanordnung wirken zur Realisierung des Schrittes S2 zusammen. Mittels einer Rekonstruktionseinheit 23 können gemäß dem Schritt S3 aus den Projektionsbildern dreidimensionale Bilddatensätze rekonstruiert werden.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Computerimplementiertes Verfahren zum Betrieb einer Röntgeneinrichtung (1) zur Aufnahme von Projektionsbildern eines Aufnahmebereichs eines Patienten (3), wobei die Röntgeneinrichtung (1) ein Stativ (4), einen Trägerarm (5), eine Halterung (9) und einen C-Bogen (10), an dem sich gegenüberliegend ein Röntgenstrahler (11) und ein Röntgendetektor (12) angeordnet sind, aufweist, wobei der Trägerarm (5) an einem Ende zur Herstellung eines ersten Bewegungsfreiheitsgrades drehbar um eine erste Rotationsachse (7) mit dem Stativ (4) gekoppelt ist und zur Herstellung eines zweiten Bewegungsfreiheitsgrades die Halterung (9) drehbar um eine zweite Rotationsachse (14) an dem anderen Ende an den Trägerarm (5) gekoppelt ist, so dass sich die erste und die zweite Rotationsachse (7, 14) sowie ein Zentralstrahl (13) des Röntgenstrahlers (11) in allen Lagen des Trägerarms (5) und der Halterung (9) in einem Punkt (17) schneiden, und wobei ferner der gebogene C-Bogen (10) in einer Führung der Halterung (9) zur Herstellung eines dritten Bewegungsfreiheitsgrades verschiebbar gelagert ist, wobei jedem der Bewegungsfreiheitsgrade ein durch eine Steuereinrichtung (17) der Röntgeneinrichtung (1) ansteuerbarer Aktor zugeordnet ist,
**dadurch gekennzeichnet, dass** die Aufnahme der Projektionsbilder entlang einer den Patienten (3) bezüglich seiner Längsrichtung lateral umgreifenden, bezüglich einer Referenzebene dreidimensionalen Lateraltrajektorie (18) des Röntgenstrahlers (11) zur Abdeckung eines Projektionswinkelbereichs von wenigstens 200° bezüglich der Referenzebene erfolgt, wobei die Referenzebene senkrecht zur Längsrichtung des Patienten (3) oder des Patiententisches (2) angeordnet ist, wobei die dreidimensionale Lateraltrajektorie (18) nicht vollständig in der Referenzebene verläuft, sondern von dieser wenigstens teilweise abweicht, wobei die Lateraltrajektorie (18) unter Ausnutzung aller drei Bewegungsfreiheitsgrade so realisiert wird, dass der C-Bogen (10) entlang der gesamten Lateraltrajektorie (18) bezüglich der Längsrichtung des Patienten (3) oder einer Patientenliege (2), auf der der Patient (3) positioniert ist, lateral neben dem Patienten (3) positioniert ist und den Patienten (3), dessen Längsrichtung und/oder dessen Längsachse seitlich umgreift.

2. Verfahren nach Anspruch 1, **dadurch gekennzeichnet, dass** die Lateraltrajektorie (18) in einem Optimierungsverfahren unter Einhaltung eines Sicherheitsabstands zu dem Patienten (3) und/oder zu Komponenten der Röntgeneinrichtung (1) als Randbedingung so ermittelt wird, dass sie möglichst nahe an einer Idealtrajektorie in der Referenzebene zu liegen kommt.

3. Verfahren nach Anspruch 2, **dadurch gekennzeichnet, dass** die Idealtrajektorie eine Teilkreisbahn ist und/oder als die Referenzebene eine vertikale, insbesondere zur Längsrichtung des Patienten (3) und/oder der Patientenliege (2) senkrechte Ebene verwendet wird.

4. Verfahren nach Anspruch 2 oder 3, **dadurch gekennzeichnet, dass** als weitere Randbedingungen wenigstens eine maximale Winkelabweichung des Zentralstrahls (13) aus der Referenzebene, insbesondere von 10 bis 20°, bevorzugt 15°, und/oder als Optimierungsziel eine Minimierung dieser Winkelabweichung vorgegeben wird.

5. Verfahren nach einem der Ansprüche 2 bis 4, **dadurch gekennzeichnet, dass** eine patientenspezifische Ausdehnungsinformation ermittelt und bei der Optimierung zur Auswertung der Randbedingungen berücksichtigt wird.

6. Verfahren nach einem der vorangehenden Ansprüche, **dadurch gekennzeichnet, dass** über einen abzudeckenden Referenzwinkelbereich, insbesondere von 200°, hinaus der Projektionswinkelbereich zur Aufnahme weiterer Projektionsbilder zur Reduzierung von Artefakten bei einer nachfolgenden Rekonstruktion erweitert wird.

7. Verfahren nach Anspruch 6, **dadurch gekennzeichnet, dass** der Projektionswinkelbereich gegenüber dem Referenzwinkelbereich um 2 bis 10°, insbesondere 5 bis 7°, erweitert ist.

8. Verfahren nach einem der vorangehenden Ansprüche, **dadurch gekennzeichnet, dass** die erste Rotationsachse (7) um 1 bis 20°, insbesondere 18°, gegenüber der Horizontalen geneigt ist und/oder der Verfahrbereich um die erste Rotationsachse (7) 140 bis 160°, insbesondere 150°, und/oder der Verfahrbereich um die zweite Rotationsachse (14) 300 bis 320°, insbesondere 310°, und/oder der Verfahrbereich durch die Verschiebung des C-Bogens (10) in der Halterung (9) 140 bis 160°, insbesondere 150°, beträgt.

9. Verfahren nach einem der vorangehenden Ansprüche, **dadurch gekennzeichnet, dass** der Röntgendetektor (12) mittels eines weiteren, durch die Steuereinrichtung (17) ansteuerbaren Aktors um eine zu dem Zentralstrahl (13) parallele, insbesondere diesem entsprechende, Detektorrotationsachse drehbar gelagert ist, wobei während der Lateraltrajektorie (18) die Steuereinrichtung (17) den weiteren Aktor so ansteuert, dass der Röntgendetektor (12) eine vorbestimmte Orientierung, insbesondere mit gegenüberliegenden Längskanten entlang einer Längsrichtung der Patientenliege (2), für alle Projektionsbilder einnimmt.

10. Verfahren nach einem der vorangehenden Ansprüche, **dadurch gekennzeichnet, dass** der Aufnahmebereich ein Abdomenbereich und/oder ein Hüftbereich ist.

11. Verfahren nach einem der vorangehenden Ansprüche, **dadurch gekennzeichnet, dass** das Stativ (4) bodenmontiert ist.

12. Verfahren nach einem der vorangehenden Ansprüche, **dadurch gekennzeichnet, dass** die Bewegung entlang der Lateraltrajektorie (18) mit einer Geschwindigkeit von wenigstens 40° pro Sekunde, insbesondere wenigstens 50° pro Sekunde, erfolgt.

13. Röntgeneinrichtung (1), aufweisend ein Stativ (4), einen Trägerarm (5), eine Halterung (9) und einen C-Bogen (10), an dem sich gegenüberliegend ein Röntgenstrahler (11) und ein Röntgendetektor (12) angeordnet sind, wobei der Trägerarm (5) an einem Ende zur Herstellung eines ersten Bewegungsfreiheitsgrades drehbar um eine erste Rotationsachse (7) mit dem Stativ (4) und zur Herstellung eines zweiten Bewegungsfreiheitsgrades die Halterung (9) drehbar um eine zweite Rotationsachse (14) an dem anderen Ende an den Trägerarm (5) angekoppelt ist, so dass sich die erste und die zweite Rotationsachse (7, 14) sowie ein Zentralstrahl (13) des Röntgenstrahlers (11) in allen Lagen des Trägerarms (5) und der Halterung (9) in einem Punkt (17) schneiden, und wobei ferner der gebogene C-Bogen (10) in einer Führung der Halterung (9) zur Herstellung eines dritten Freiheitsgrades verschiebbar gelagert ist, wobei zu jedem der Freiheitsgrade ein durch eine Steuereinrichtung (17) der Röntgeneinrichtung (1) ansteuerbarer Aktor zugeordnet ist, **dadurch gekennzeichnet, dass** die Steuereinrichtung (17) zur Durchführung eines Verfahrens nach einem der vorangehenden Ansprüche ausgebildet ist.

## Claims

1. Computer-implemented method for operating an X-ray device (1) for acquiring projection images of an acquisition region of a patient (3), wherein the X-ray device (1) has a stand (4), a support arm (5), a mount (9) and a C-arm (10), on which an X-ray generator (11) and an X-ray detector (12) are arranged, opposing one another, wherein the support arm (5) is coupled to the stand (4) at one end such that it can rotate about a first axis of rotation (7) for the creation of a first degree of freedom of movement and the mount (9) is coupled to the support arm (5) at the other end such that it can rotate about a second axis of rotation (14) for the creation of a second degree of freedom of movement, so that the first and the second axis of rotation (7, 14) as well as a central beam (13) of the X-ray generator (11) intersect at one point (17) in all positions of the support arm (5) and of the mount (9), and wherein the curved C-arm (10) is further displaceably mounted in a guide of the mount (9) for the creation of a third degree of freedom of movement, wherein each of the degrees of freedom of movement is assigned an actuator that can be activated by a control device (17) of the X-ray device (1),
**characterised in that** the acquisition of the projection images takes place along a lateral trajectory (18), three-dimensional in respect of a reference plane, of the X-ray generator (11) encompassing the patient (3) laterally in respect of the latter's longitudinal direction, for the coverage of a projection angle range of at least 200° in respect of the reference plane, wherein the reference plane is arranged perpendicularly to the longitudinal direction of the patient (3) or the patient couch (2), wherein the three-dimensional lateral trajectory (18) does not run completely in a reference plane, but deviates therefrom at least in part, wherein the lateral trajectory (18) is implemented using all three degrees of freedom of movement so that the C-arm (10) is positioned laterally next to the patient (3) along the entire lateral trajectory (18) in respect of the longitudinal direction of the patient (3) or of a patient couch (2) on which the patient (3) is positioned and encompasses the patient (3), the longitudinal direction thereof and/or the longitudinal axis thereof laterally.

2. Method according to claim 1, **characterised in that** the lateral trajectory (18) is determined in an optimisation procedure while maintaining a safe distance from the patient (3) and/or from components of the X-ray device (1) as a boundary condition, so that they come to lie as close as possible to an ideal trajectory in the reference plane.

3. Method according to claim 2, **characterised in that** the ideal trajectory is a partial circular path, and/or a vertical plane, in particular perpendicular to the longitudinal direction of the patient (3) and/or of the patient couch (2), is used as the reference plane.

4. Method according to claim 2 or 3, **characterised in that** as further boundary conditions at least a maximum angular deviation of the central beam (13) from the reference plane, in particular from 10 to 20°, preferably 15°, is specified and/or a minimisation of said angular deviation is specified as an optimisation target.

5. Method according to one of claims 2 to 4, **characterised in that** patient-specific expansion information is determined and is taken into account during the optimisation for the evaluation of the boundary conditions.

6. Method according to one of the preceding claims, **characterised in that** the projection angle range for the acquisition of further projection images for the reduction of artifacts in a subsequent reconstruction is extended beyond a reference angle range to be covered, in particular of 200°.

7. Method according to claim 6, **characterised in that** the projection angle range is extended compared to the reference angle range by 2 to 10°, in particular 5 to 7°.

8. Method according to one of the preceding claims, **characterised in that** the first axis of rotation (7) is inclined by 1 to 20°, in particular 18°, compared to the horizontal and/or the travel range about the first axis of rotation (7) is 140 to 160°, in particular 150°, and/or the travel range about the second axis of rotation (14) is 300 to 320°, in particular 310°, and/or the travel range due to the displacement of the C-arm (10) in the mount (9) is 140 to 160°, in particular 150°.

9. Method according to one of the preceding claims, **characterised in that** the X-ray detector (12) is mounted such that it can rotate by means of a further actuator, which can be activated by the control device (17), about a detector axis of rotation parallel to the central beam (13), in particular corresponding thereto, wherein during the lateral trajectory (18) the control device (17) activates the further actuator so that the X-ray detector (12) assumes a specified orientation, in particular with opposing longitudinal edges along a longitudinal direction of the patient couch (2), for all projection images.

10. Method according to one of the preceding claims, **characterised in that** the acquisition region is an abdominal region and/or a hip region.

11. Method according to one of the preceding claims, **characterised in that** the stand (4) is floor-mounted.

12. Method according to one of the preceding claims, **characterised in that** the movement along the lateral trajectory (18) takes place at a speed of at least 40° per second, in particular at least 50° per second.

13. X-ray device (1), having a stand (4), a support arm (5), a mount (9) and a C-arm (10), on which are arranged, opposing one another, an X-ray generator (11) and an X-ray detector (12), wherein the support arm (5) is coupled to the stand (4) at one end such that it can rotate about a first axis of rotation (7) for the creation of a first degree of freedom of movement and the mount (9) is coupled to the support arm (5) at the other end such that it can rotate about a second axis of rotation (14) for the creation of a second degree of freedom of movement, so that the first and the second axis of rotation (7, 14) as well as a central beam (13) of the X-ray generator (11) intersect at one point (17) in all positions of the support arm (5) and of the mount (9), and wherein the curved C-arm (10) is further displaceably mounted in a guide of the mount (9) for the creation of a third degree of freedom, wherein each of the degrees of freedom is assigned an actuator that can be activated by a control device (17) of the X-ray device (1), **characterised in that** the control device (17) is designed for the performance of a method according to one of the preceding claims.

## Revendications

1. Procédé mis en œuvre par ordinateur pour faire fonctionner un dispositif (1) à rayons X d'enregistrement d'images de projection d'une partie à enregistrer d'un patient (3), dans lequel le dispositif (1) à rayons X a un statif (4), un bras (5) porteur, une fixation (9) et un arceau (10) en C, sur lequel sont montés, en se faisant face, un émetteur (11) de rayons X et un détecteur (12) de rayons X, dans lequel le bras (5) porteur est, à une extrémité, accouplé au statif (4) en rotation autour d'un premier axe (7) de rotation pour la production d'un premier degré de liberté de mouvement et pour la production d'un deuxième degré de liberté de mouvement, la fixation (9) est, à l'autre extrémité du bras (5) porteur, accouplée en rotation autour d'un deuxième axe (14) de rotation de sorte que le premier et le deuxième axes (7, 14) de rotation ainsi qu'un rayon (13) central de l'émetteur (11) de rayons X se coupent en un point (17) en toutes les positions du bras porteur (5) et de la fixation (9) et dans lequel en outre l'arceau (10) en C incurvé est monté coulissant dans un guidage de la fixation (9) pour la production d'un troisième degré de liberté de mouvement, dans lequel un actionneur pouvant être commandé par un dispositif (17) de commande du dispositif (1) à rayons X est affecté à chacun des degrés de liberté de mouvement, **caractérisé en ce que** l'enregistrement des images de projection s'effectue, pour couvrir une plage d'angle de projection d'au moins 200° par rapport au plan de référence, le long d'une trajectoire (18) latérale, en trois dimensions par rapport au plan de référence et entourant latéralement le patient (3) rapporté à sa direction longitudinale, de l'émetteur (11) de rayons X, dans lequel le plan de référence est perpendiculaire à la direction longitudinale du patient (3) ou de la couchette (2) pour le patient, dans lequel la trajectoire (18) latérale en trois dimensions ne s'étend pas complètement dans le plan de référence, mais s'en écarte au moins en partie, dans lequel on réalise la trajectoire (18) latérale est réalisée en utilisant tous les trois degrés de liberté de mouvement, de manière à ce que l'arceau en C (10) soit mis en position, le long de toute la trajectoire (18) latérale, rapportée à la direction longitudinale du patient (3) ou d'une couchette (2) de patient, sur laquelle le patient (3) est mis en position, latéralement à côté du patient (3) et entoure latéralement le patient (3), sa direction longitudinale et/ou son axe longitudinal.

2. Procédé suivant la revendication 1, **caractérisé en ce que** l'on détermine la trajectoire (18) latérale dans un procédé d'optimisation en respectant une distance de sécurité au patient (3) et/ou à des composants du dispositif (1) à rayons X comme condition au limite, de manière à ce qu'elle se rapproche le plus possible d'une trajectoire idéale dans le plan de référence.

3. Procédé suivant la revendication 2, **caractérisé en ce que** la trajectoire idéale est une orbite circulaire partielle et/ou **en ce que** l'on utilise, comme plan de référence, un plan vertical, en particulier perpendiculaire à la direction longitudinale du patient (3) et/ou de la couchette (2) pour le patient.

4. Procédé suivant la revendication 2 ou 3, **caractérisé en ce que** l'on prescrit comme autres conditions aux limites au moins un écart angulaire maximum du rayon (13) central hors du plan de référence, en particulier de 10 à 20° et de préférence de 15° et/ou comme cible d'optimisation une minimisation de cet écart angulaire.

5. Procédé suivant l'une des revendications 2 à 4, **caractérisé en ce que** l'on détermine une information d'étendue spécifique au patient et on en tient compte lors de l'optimisation pour l'évaluation des conditions aux limites.

6. Procédé suivant l'une des revendications précédentes, **caractérisé en ce qu'**au-delà d'une plage angulaire de référence à couvrir, en particulier de 200°, on agrandit la plage angulaire de projection pour l'enregistrement d'autres images de projection afin de réduire des artefacts lors d'une reconstruction venant ensuite.

7. Procédé suivant la revendication 6, **caractérisé en ce que** la plage angulaire de projection est agrandie de 2 à 10°, en particulier de 5 à 7°, par rapport à la plage angulaire de référence.

8. Procédé suivant l'une des revendications précédentes, **caractérisé en ce que** le premier axe (7) de rotation est incliné de 1 à 20°, en particulier de 18°, par rapport à l'horizontal et/ou la plage de mouvement autour du premier axe (7) de rotation va de 140 à 160°, en étant en particulier de 150°, et/ou la plage de mouvement autour du deuxième axe (14) de rotation va de 300 à 320°, en étant en particulier de 310°, et/ou la plage de mouvement par le coulissement de l'arceau en C (10) dans la fixation (9) va de 140 à 160°, en étant en particulier de 150°.

9. Procédé suivant l'une des revendications précédentes, **caractérisé en ce que** le détecteur (12) de rayons X est, au moyen d'un autre actionneur pouvant être commandé par le dispositif (17) de commande, monté tournant autour d'un axe de rotation de détecteur parallèle au rayon (13) central, en particulier correspondant à celui-ci, dans lequel pendant la trajectoire (18) latérale, le dispositif (17) de commande commande l'autre actionneur, de manière à ce que le détecteur (12) de rayons X prenne, pour toutes les images de projection, une orientation déterminée à l'avance, en particulier par des bords longitudinaux opposés le long d'une direction longitudinale de la couchette (2) pour le patient.

10. Procédé suivant l'une des revendications précédentes, **caractérisé en ce que** la partie à enregistrer est une partie de l'abdomen et/ou une partie de la hanche.

11. Procédé suivant l'une des revendications précédentes, **caractérisé en ce que** le statif (4) est monté sur le sol.

12. Procédé suivant l'une des revendications précédentes, **caractérisé en ce que** le mouvement le long de la trajectoire (18) latérale s'effectue à une vitesse d'au moins 40° à la seconde, en particulier d'au moins 50° à la seconde.

13. Dispositif (1) à rayons X, comportant un statif (4) un bras (5) porteur, une fixation (9) et un arceau (10) en C, sur lequel sont montés, en se faisant face, un émetteur (11) de rayons X et un détecteur (12) de rayons X, dans lequel le bras (5) porteur est, à une extrémité, pour la production d'un premier degré de liberté de mouvement, accouplé au statif (4) en rotation autour d'un premier axe (7) de rotation et, pour la production d'un deuxième degré de liberté de mouvement, la fixation (9) est accouplée en rotation autour d'un deuxième axe (14) de rotation à l'autre extrémité du bras (5) porteur, de sorte que le premier et le deuxième axes (7, 14) de rotation ainsi qu'un rayon (13) central de l'émetteur (11) de rayons X se coupent en un point (17) en toutes les positions du bras porteur (5) et de la fixation (9) et dans lequel en outre l'arceau (10) en C incurvé est monté coulissant dans un guidage de la fixation (9) pour la production d'un troisième degré de liberté de mouvement, dans lequel un actionneur pouvant être commandé par un dispositif (17) de commande du dispositif (1) à rayons X est affecté à chacun des degrés de liberté de mouvement, **caractérisé en ce que** le dispositif (17) de commande est constitué pour effectuer un procédé suivant l'une des revendications précédentes.
